(19) Europäisches Patentamt
European Patent Office
Office européen des brevets

(11) Numéro de publication: **0 149 583**
**B1**

# (12) FASCICULE DE BREVET EUROPÉEN

(45) Date de publication du fascicule du brevet: **31.05.89**

(51) Int. Cl.⁴: **C 07 D 405/12**, C 07 D 409/12, A 61 K 31/44

(21) Numéro de dépôt: **85400085.8**

(22) Date de dépôt: **18.01.85**

## (54) 3-Alkoxy-2(N-pyrrolidino)-N-pyridyl-N-furyl (ou thienyl)-methyl-propylamines, leur préparation et les compositions pharmaceutiques les contenant.

(30) Priorité: **18.01.84 FR 8400755**

(43) Date de publication de la demande: **24.07.85 Bulletin 85/30**

(45) Mention de la délivrance du brevet: **31.05.89 Bulletin 89/22**

(84) Etats contractants désignés: **AT BE CH DE FR GB IT LI LU NL SE**

(56) Documents cité: **FR-A-2 378 024**

**A. BURGER: "Medicinal chemistry", édition 3, partie I, 1970, pages 74-77, John Wiley & Sons, New York, US;**

**Le dossier contient des informations techniques présentées postérieurement au dépôt de la demande et ne figurant pas dans le présent fascicule.**

(73) Titulaire: **RIOM LABORATOIRES- C.E.R.M. "R.L.-CERM" - (S.A.), Route de Marsat B.P. 140, F-63203 Riom Cédex (FR)**

(72) Inventeur: **Combourieu, Michel, 6, rue du Mont Mouchet, F-15000 Aurillac (FR)**
Inventeur: **Simond, Jacques, Rue des Thuilets Mirefleurs, F-63730 Les- martres- de- veyre (FR)**
Inventeur: **Monteil, André, Route de Prompsat Les Grosliers, F-63140 Chatel- Guyon (FR)**

(74) Mandataire: **Hermans, Franciscus G.M., Patent Department AKZO N.V. Pharma Division P.O. Box 20, NL- 5340 BH Oss (NL)**

**EP 0 149 583 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Beschreibung**

La présente invention a pour objet de nouvelles 3-alkoxy-2-(N-pyrrolidino)-N-pyridyl-N-furylméthyl propylamines et des analogues N-thiénylméthyl correspondants, des méthodes pour leur préparation et une composition pharmaceutique les contenant.

Les composés correspondent plus particulièrement à la formule générale suivante:

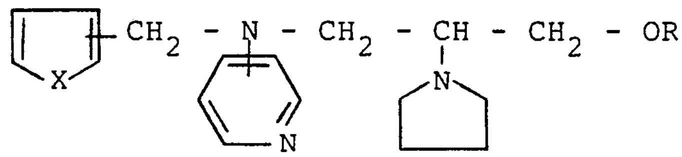

(I)

dans laquelle X représente un atome d'oxygène ou de soufre et R représente un radical alkyle linéaire ou ramifié de 1 à 7 atomes de carbone, et leurs sels d'addition acide pharmaceutiquement acceptables.

On connaît déjà plusieurs propylamines substituées, en particulier le composé du brevet FR-A-2 378 034 de formule

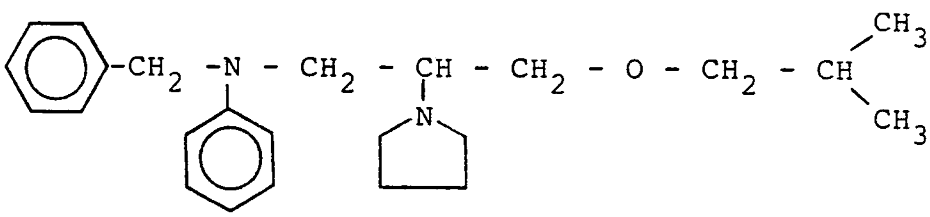

décrit en particulier pour son activité antiangineuse.

Par rapport à ce composé, les composés de l'invention se caractérisent, sur le plan chimique, par remplacement des deux noyaux phényle par des noyaux hétérocycliques entraînant, sur le plan de l'activité, une activité antiarythmique venant en complément de leurs activités antiangineuse et antihypertensive, activité antiarythmique que ne possède pas à un tel dégré le composé de l'art antérieur.

Les composés de formule I peuvent être préparés par des méthodes connues pour la préparation de composés analogues. Les composés (I) peuvent être préparés par la réaction du composé de formule II

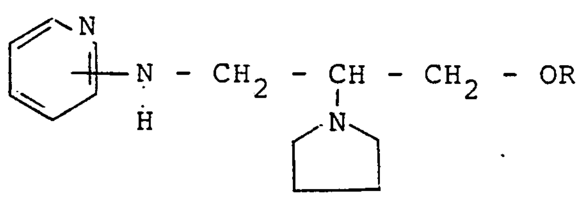

(II)

avec un dérivé halogéné de formule III

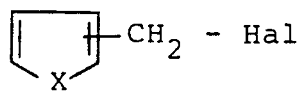

(III)

dans laquelle R et X ont les significations précédemment indiquées et Hal représente un atome d'halogène, de préférence le chlore ou le brome.

Le composé de départ de formule II peut par exemple être préparé par réaction d'un composé de formule IV

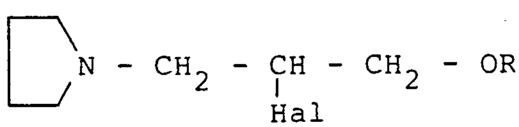

(IV)

dans laquelle Hal et R ont les significations précédemment indiquées, avec l'aminopyridine.

Cette réaction se déroule de préférence en traitant d'abord l'aminopyridine appropriée par un agent de métallation alcalin tel que l'hydrure ou l'amidure de sodium.

Une autre méthode pour la préparation des composés de formule I consiste à faire réagir un composé de

formule IV avec un composé de formule V

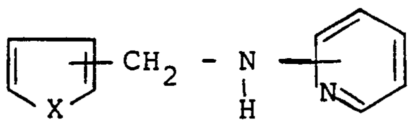

(V)

dans laquelle X a les significations précédemment indiquées. De préférence la réaction d'un composé IV avec un composé V est facilitée en effectuant d'abord une métallation du dernier composé au moyen d'un agent de métallation alcalin tel que l'hydrure de sodium, ou l'amidure de sodium, potassium ou lithium.

Le composé de formule V peut être obtenu par réaction d'une aldéhyde, furfuraldéhyde ou son analogue soufré correspondant, avec l'aminopyridine pour donner une imine qui est ensuite réduite in situ pour donner l'amide secondaire désirée de formule V.

Un agent de réduction approprié est par exemple le borohydrure de sodium.

Les composés de formule I et leurs sels d'addition acide se sont révélés posséder d'intéressantes propriétés anticalciques mises en évidence in vitro par les méthodes usuelles de pharmacologie moléculaire [VAN ROSSUM Arch. Int. Pharmacodyn. Ther. 143, 299 - 330, (1983)].

In vivo, on a recherché l'activité antiangineuse en mesurant chez le chien anesthésié les paramètres hémodynamiques habituels (pourcentage de variation et durée d'action):

- Fréquence cardiaque à l'aide d'électrodes à E C G sous-cutannées.
- Débit artériel coronaire à l'aide d'un débitmètre électromagnétique.
- Action antitachycardisante (inhibition des effets chronotropes positifs de l'isoprénaline).

Les composés de l'invention sont administrés par voie I.V. à la dose de 5 $mg.kg^{-1}$ et les résultats rapportés dans le tableau I ci-après.

**Tableau I**

| | | Exemple 1 | Exemple 2 |
|---|---|---|---|
| Fréquence cardiaque | Variation % | - 19 % | - 22 % |
| | Durée (mn) | > 45 mn | >45 mn |
| Débit coronaire | Variation % | + 97 % | + 65 % |
| | Durée (mn) | 5 mn | 5 mn |
| Action anti-tachycardisante | Variation % | - 63 % | - 63 % |
| | Durée (mn) | 45 mn | 45 mn |

Ces résultats font apparaître pour les composés de l'invention des effets bradycardisants et antitachycardisants, ainsi qu'un effet coronarodilatateur notable.

Les composés de l'invention se sont en outre révélés posséder une toxicité réduite: leur toxicité aiguë par voie orale chez la souris est généralement supérieure à 500 $mg.kg^{-1}$.

Cet ensemble de propriétés pharmacologiques permet donc d'envisager l'application des composés de l'invention en thérapeutique en tant que médicament pour le traitement des troubles cardiovasculaires tels que l'angine de poitrine, l'hypertension ou les troubles du rythme. Associés aux excipients pharmaceutiques habituels, ils pourront être administrés par voie orale ou intraveineuse, à des doses quotidiennes comprises entre 1 et 15 mg par kg de poids corporel. En thérapeutique humaine, on utilisera une posologie comprise entre 40 et 1200 mg, et de préférence entre 100 et 800 mg.

Mélangés avec des excipients convenables, les composés I, ou un de leurs sels, peuvent être comprimés sous forme de prise unitaire tels que comprimés, pilules etc... ou peuvent être mis en gélules. Au moyen de liquides convenables, les composés peuvent aussi être utilisés en préparations injectables ou orales sous forme de solutions, suspensions ou émulsions.

Les composés de formule I possèdent un atome de carbone asymétrique de sorte qu'il est possible d'obtenir un mélange racémique de I et les énantiomères optiques séparés. Le mélange racémique et les énantiomères optiques séparés appartiennent à la fois aux composés de l'invention.

Les énantiomères optiques peuvent être séparés de manière habituelle par résolution du mélange racémique, ou directement en partant de matières premières optiquement actives.

L'invention concerne aussi les sels d'addition acide des composés de formule I pharmaceutiquement acceptables. Ces sels sont obtenus de manière habituelle en combinant la base libre I avec des acides inorganiques ou organiques, tels que les acides chlorhydrique, fumarique, maléique, citrique ou succinique, ces acides n'étant mentionnés qu'à titre illustratif mais sans impliquer de limitation.

Le groupe alkyle dans la définition de R est un groupe alkyle linéaire ou ramifié de 1 à 7 atomes de carbone, tels que les radicaux méthyle, éthyle, propyle, butyle, isobutyle, pentyle, isopentyle, et hexyle. Pour la définition de R, le radical isobutyle est le groupe préféré.

Le groupe 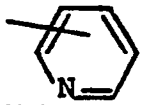 comprend tous les radicaux pyridyles dont le radical 2-pyridyle constitue une réalisation préférée.

Les groupes furyle et thiényle dans les composés de formule I comprennent les radicaux 2-furyle, 3-furyle, 2-thiényle et 3-thiényle. Les radicaux 2-furyle et spécialement 2-thiényle constituent les réalisations préférées.

Les composés préférés selon l'invention sont les composés de formule I et leurs sels d'addition acide, dans lesquels seuls ou en combinaison, R représente le

reste isobutyle, le groupe 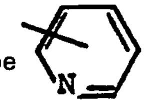 représente le reste

2-pyridyle et le groupe 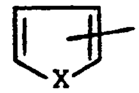 représente un reste 2-furyle ou 2-thiényle.

**Exemple 1**

3-isobutoxy 2-(N-pyrrolidino)-N-(2-pyridyl) N-(2-thiényl) méthyl propylamine

Dans une première étape, on a introduit dans un tricol de 2 litres sous agitation magnétique chauffante 20 g (0,21 M) de 2-amino pyridine dans 400 ml de toluène puis 7,5 g (0,25 M) de NaH à 80 % et porté le mélange 1/2 h à reflux. Après avoir laissé revenir à la température ambiante, on a introduit 46,15 g (0,21 M) de 2-chloro-1-isobutoxy-3-pyrrolidino propane et porté 3 heures à reflux. Après avoir laissé revenir à température ambiante, on a lavé à l'eau, séché sur $MgSO_4$, filtré, évaporé le solvant à sec et distillé le résidu sous pression réduite.

On a ainsi obtenu 38,8 g de 3-isobutoxy-2-(N-pyrrolidino)-N-(2-pyridyl) propylamine ayant pour point d'ébullition $E_{0,05}$ = 154°C.

Dans une deuxième étape, on a introduit 14 g (0,053 M) de l'amine précédente dans un tricol contenant 250 ml de toluène, puis 2 g (0,067 M) de NaH et porté une 1/2 heure à reflux. Après avoir laissé revenir à température ambiante, on a ajouté 7,3 g (0,055 M) de 2-chloro méthyl thiophène et porté le mélange 3 heures à reflux. Après avoir laissé revenir à température ambiante, on a lavé, séché sur $MgSO_4$, filtré, évaporé le solvant et distillé le résidu sous pression réduite.

On a ainsi obtenu 4 g de composé du titre ayant pour point d'ébullition $E_{0,05}$ = 180°C. Le point de fusion de l'hémifumarate est de 99,8°C.

**Exemple 2**

3-isobutoxy-2-(N-pyrrolidino)-N-(2-pyridyl)-N-(2-furyl) méthyl propylamine

Dans une première étape, on a porté au reflux azéotropique pendant 2 heures 20 g (0,213 M) de 2-amino pyridine et 22,5 g (0,234 M) de furfuraldéhyde dans 400 ml de benzène. on a ensuite évaporé le benzène, repris par 500 ml de méthanol, mis le mélange sous agitation au bain de glace et introduit par petites portions 8,9 g (0,234 M) de $NaBH_4$, puis on a maintenu sous agitation à température ambiante pendant 5 heures. Après avoir évaporé le méthanol, on a extrait à l'éther et obtenu 24,1 g de 2-(2-furyl)-méthyl-amino-pyridine ayant pour point d'ébullition $E_{0,1}$ = 98°C.

Dans une deuxième étape, on a introduit 12 g (0,068 M) de l'amine précédente dans un ballon contenant 200 ml de toluène puis 2,8 g (0,092 M) de NaH à 80 %, puis porté 1/2 heure à reflux, laissé revenir à température ambiante, puis introduit 14,9 g (0,068 M) de 2-chloro 1-isobutoxy-3-pyrrolidino propane et porté à reflux jusqu'à disparition du dérivé aminé. On a ensuite détruit l'excès de NaH avec un peu d'eau saturée en NaCl, lavé à l'eau, séché sur $MgSO_4$, filtré, évaporé le toluène et distillé le résidu sous pression réduite. On a ainsi obtenu 8,6 g de composé du titre ayant pour point d'ébullition $E_{0,05}$ = 169°C.

**Patentansprüche**

1. Composés de formule générale I

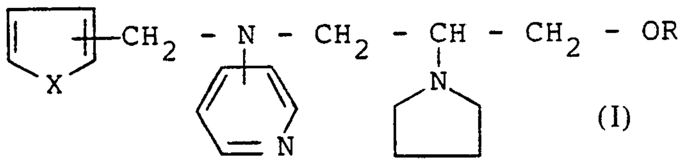

(I)

dans laquelle X représente un atome d'oxygène ou le soufre et R représente un radical alkyle linéaire ou ramifié de 1 à 7 atomes de carbone, et leurs sels d'addition acide pharmaceutiquement acceptables.

2. Composé selon la revendication 1 caractérisé en ce que R représente le radical isobutyle.

3. Composé selon la revendication 1 caractérisé en ce que le groupe 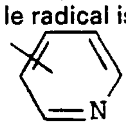 représente le radical 2-pyridyle.

4. Composé selon la revendication 1 caractérisé en ce que le groupe 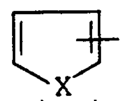 représente le radical 2-thiényle.

5. Composition pharmaceutique caractérisée en ce qu'elle contient à titre de principe actif au moins un des composés selon la revendication 1 associée à un ou plusieurs excipients convenables.

6. Procédé pour la préparation des composés selon la revendication 1 caractérisé en ce qu'on fait réagir un composé de formule II

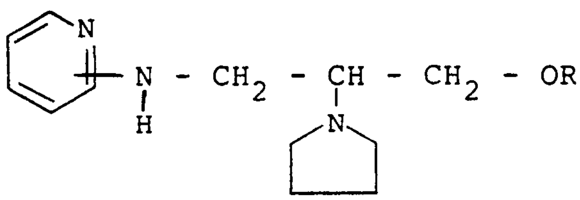

(II)

avec un dérivé halogéné de formule III

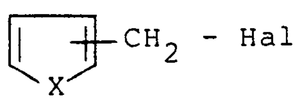

(III)

dans lesquelles X et R ont les significations de la revendication 1 et Hal désigne un atome d'halogène, et qu'on peut ensuite transformer le composé de formule I obtenu en un sel d'addition acide pharmaceutiquement acceptable.

7. Procédé pour la préparation des composés selon la revendication 1 caractérisé en ce qu'on fait réagir un composé de formule IV

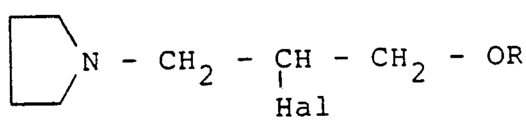

(IV)

avec une amine de formule V

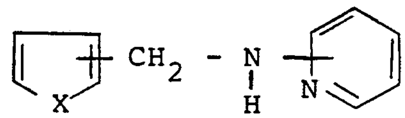

(V)

dans lesquelles X et R ont les significations de la revendication 1 et Hal désigne un atome d'halogène, et qu'on peut ensuite transformer le composé de formule I obtenu en un sel d'addition acide pharmaceutiquement acceptable.

**Patentansprüche**

1. Verbindungen der allgemeinen Formel I

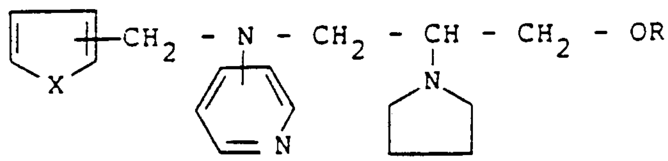

(I)

worin X für ein Sauerstoff- oder Schwefelatom und R für einen geradkettigen oder verzweigten Alkylrest mit 1 bis 7 Kohlenstoffatomen stehen, sowie deren pharmazeutisch unbedenkliche Säureadditionssalze.

2. Verbindungen nach Anspruch 1, dadurch gekennzeichnet, daß R für den Isobutylrest steht.

3. Verbindungen nach Anspruch 1, dadurch gekennzeichnet, daß die Gruppe 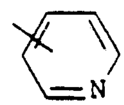 für den 2-Pyridylrest steht.

4. verbindungen nach Anspruch 1, dadurch gekennzeichnet, daß die Gruppe 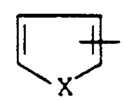 für den 2-Thienylrest steht.

5. Pharmazeutische Zusammensetzung, dadurch gekennzeichnet, daß sie als Wirkstoff mindestens eine der Verbindungen nach Anspruch 1 zusammen mit einem oder mehreren geeigneten Trägern enthält.

6. Verfahren zur Herstellung von Verbindungen nach Anspruch 1, dadurch gekennzeichnet, daß man eine Verbindung der Formel II

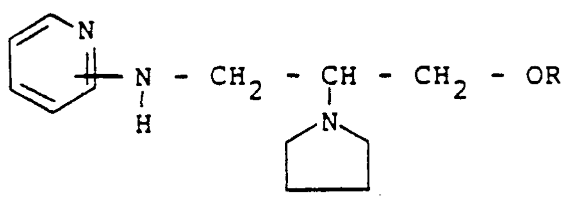

(II)

mit einem halogenierten Derivat der Formel III

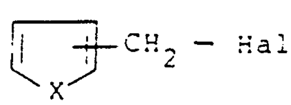

(III)

worin X und R die in Anspruch 1 angegebenen Bedeutungen haben und Hal ein Halogenatom bezeichnet, umsetzt und gegebenenfalls anschließend die so erhaltene Verbindung der Formel I in ein pharmazeutisch unbedenkliches Säureadditionssalz überführt.

7. Verfahren zur Herstellung von Verbindungen nach Anspruch 1, dadurch gekennzeichnet, daß man eine Verbindung der Formel IV

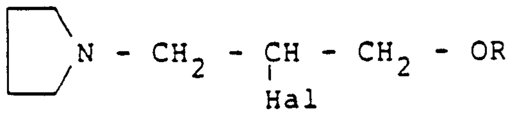 (IV)

mit einem Amin der Formel V

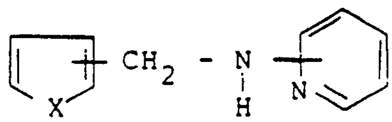 (V)

worin X und R die in Anspruch 1 angegebenen Bedeutungen haben und Hal ein Halogenatom bezeichnet, umsetzt und gegebenenfalls anschließend die so erhaltene Verbindung der Formel I in ein pharmazeutisch unbedenkliches Säureadditionssalz überführt.

## Claims

Compounds of general formula I

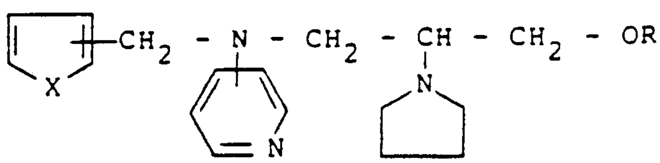 (I)

in which X denotes an oxygen or sulphur atom and R denotes a linear or branched alkyl radical having 1 to 7 carbon atoms, and their pharmaceutically acceptable addition salts formed with an acid.

2. Compound according to Claim 1, characterized in that R denotes an isobutyl radical.

3 Compound according to Claim 1, characterised in that the group 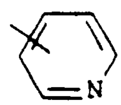 denotes a 2-pyridyl radical.

4. Compound according to Claim 1, characterized in that the group 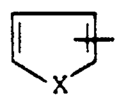 denotes a 2-thienyl radical.

5. Pharmaceutical composition, characterized in that it contains, by way of active principal, at least one of the compounds according to Claim 1, in combination with one or more suitable excipients.

6. Process for the preparation of the compounds according to Claim 1, characterized in that a compound of formula II

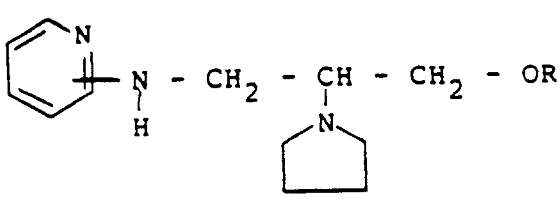 (II)

is reacted with a halogenated derivative of formula III

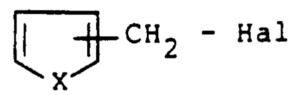 (III)

in which formulae X and R have the meanings of Claim 1 and Hal denotes a halogen atom,

and in that the compound of formula I obtained can then be converted to a pharmaceutically acceptable addition salt formed with an acid.

7. Process for the preparation of the compounds according to Claim 1, characterized in that a compound of formula IV

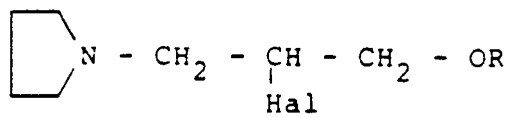 (IV)

is reacted with an amine of formula V

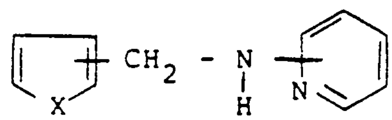 (V)

in which formulae X and R have the meanings of Claim 1 and Hal denotes a halogen atom,

and in that the compound of formula I obtained can then be converted to a pharmaceutically acceptable addition salt formed with an acid.